# EUROPEAN PATENT SPECIFICATION

(11) **EP 4 035 617 B1**
(45) Date of publication and mention of the grant of the patent: **20.08.2025**
(21) Application number: 20872765.1
(22) Date of filing: 29.09.2020
(51) Int. Cl.: A61B 18/14

(54) **MEDICAL DEVICE**
MEDIZINISCHE VORRICHTUNG
DISPOSITIF MÉDICAL

(30) Priority: 30.09.2019 JP 2019178331
(43) Date of publication of application: 03.08.2022
(73) Proprietor: TERUMO Kabushiki Kaisha, Tokyo 151-0072 (JP)
(72) Inventor: TAKAHASHI, Yusuke, Ashigarakami-gun, Kanagawa 259-0151 (JP)
(74) Representative: Casalonga
(86) International application number: PCT/JP2020/036816
(87) International publication number: WO 2021/065874

(56) References cited:
- EP-A1- 3 766 431
- EP-A1- 3 777 740
- EP-A1- 3 777 741
- WO-A1-2019/009254
- WO-A1-2019/009254
- WO-A1-2019/085841
- WO-A1-2019/109013
- WO-A1-2019/179447
- JP-A- 2012 050 538
- JP-A- 2014 512 869

## Description

### Technical Field

This disclosure relates to a medical device for applying energy to a biological tissue. In particular, the present invention relates to a medical device according to the preamble of claim 1, such as it is e.g. known from WO2019009254A1, EP 3766431 A1, WO 2019 109013 A1 or WO 2019 085841 A1.

### Background Art

Chronic heart failure is a known heart disease. Chronic heart failure is broadly classified into a systolic heart failure and a diastolic heart failure, based on a cardiac function index. In a patient suffering from the diastolic heart failure, myocardial hypertrophy appears, and stiffness (hardness) increases, so that blood pressure increases in a left atrium, and a cardiac pumping function is degraded. In this manner, the patient may show heart failure symptoms such as a pulmonary edema. In addition, there is another heart disease of a patient who shows the heart failure symptom because blood pressure increases on a right atrium side due to pulmonary hypertension, and the cardiac pumping function is degraded.

In recent years, shunt treatments have attracted attention in which, for the patients who suffer from heart failure, a shunt (through-hole) serving as an escape route for increased atrial pressure is formed in an atrial septum, thereby enabling heart failure symptoms to be alleviated. In the shunt treatment, the atrial septum is accessed using an intravenous approaching method, and the through-hole is formed to have a desired size. For example, a medical device disclosed in PTL 1 is used as one of medical devices for performing the shunt treatment on the atrial septum.

### Citation List

### Patent Literature

PTL 1: US 8882697

### Summary of Invention

### Technical Problem

In the medical device of PTL 1, a shunt hole is enlarged using a balloon serving as an expansion body that is provided in a distal portion of a shaft portion, and the shunt hole is maintained by an electrode provided in the balloon. However, in this medical device, the electrode (energy transfer element) is exposed to blood, so that energy is provided into the blood and an unintended site, which may cause variations in the degree of cauterization, formation of a thrombus, generation of tissue damage and the like.

This disclosure is made in order to solve the above-described problem, and an object thereof is to provide a medical device that can reduce variations in the degree of cauterization by an energy transfer element, and can suppress generation of thrombus formation, tissue damage, and the like due to the cauterization.

### Solution to Problem

In order to solve the above problem the present invention provides a medical device according to claim 1. The dependent claims relate to advantageous embodiments.

### Advantageous Effect of Invention

With the medical device configured as the above, the receiving surface is approximately parallel to the energy transfer element in accordance with the movement of the energy transfer element, so that the energy transfer element can come into close contact with the biological tissue that is clamped between the energy transfer element and the receiving surface. Therefore, variations in the degree of cauterization by the energy transfer element can be reduced. Moreover, the energy transfer element can be prevented from locally floating from the biological tissue. Therefore, the energy transfer element is prevented from supplying energy into blood and an unintended site, and generation of thrombus formation, tissue damage, and the like due to the cauterization can be suppressed.

The at least one clamping portion may include two outer peripheral portions on both sides in a width direction that is a direction orthogonal to an axis direction of the expansion body, and a direction orthogonal to the radial direction of the expansion body, relative to the back support portion. In this manner, the outer peripheral portions effectively guide the energy transfer element that moves toward the back support portion, to the back support portion that is positioned between the outer peripheral portions. Therefore, the energy transfer element can press the biological tissue supported by the two outer peripheral portions, and can press the biological tissue against the back support portion that is disposed between the two outer peripheral portions. Therefore, the energy transfer element comes into close contact with the biological tissue and is hard to float from the biological tissue, and the position of the energy transfer element relative to the biological tissue is stably maintained by the two outer peripheral portions.

The two outer peripheral portions each may have a convex shape to an outer side in the width direction. In this manner, between the two outer peripheral portions, a wide region in which the back support portion is disposed can be secured. Moreover, the two outer peripheral portions in the width direction can support the biological tissue in the wide range, so that the energy transfer element and the receiving surface that clamp the biological tissue therebetween between the two outer peripheral portions are easily maintained in the suitable positions.

The two outer peripheral portions each may have a circular arc shape that smoothly projects to the outer side in the width direction. In this manner, the outer peripheral portions is configured to be stored in an inner surface of a storage sheath without being caught thereon, which is a tubular member that stores the expansion body so as to be releasable, for example. Accordingly, the outer peripheral portions can be smoothly stored in the storage sheath, and can be smoothly released from the storage sheath.

Moreover, a maximum width of the outer peripheral portions that sandwich the back support portion therebetween in the width direction may be larger than a maximum width of the energy transfer element in the width direction. In this manner, the outer peripheral portion easily guides a press direction of the energy transfer element toward the back support portion.

The back support portion may move larger than the two outer peripheral portions due to a force in the axis direction to be received from the energy transfer element. In this manner, the back support portion can flexibly receive the biological tissue that is pressed by the energy transfer element while moving rearward larger in the press direction of the energy transfer element than the outer peripheral portion. Therefore, the energy transfer element comes into close contact with the biological tissue and is hard to float from the biological tissue.

The expansion body may include an inner projection portion between the energy transfer element and the back support portion, and the maximum width between the two outer peripheral portions that sandwich the back support portion therebetween in the width direction may be larger than a maximum width of the inner projection portion in the width direction. In this manner, while maintaining the flexibility of the inner projection portion, a structure in which the width between the two outer peripheral portions is widened can be obtained.

The back support portion may have a cantilever beam shape that extends from the wire portion. In this manner, the back support portion can warp flexibly by receiving a force.

The back support portion may be a member that is supported by at least one flexible support wire that extends from the two outer peripheral portions that sandwich the back support portion therebetween. In this manner, the back support portion can move lager than the outer peripheral portions due to a force in the axis direction.

The back support portion may be at least one flexible back support wire that extends from the two outer peripheral portions that sandwich the back support portion therebetween. In this manner, the back support portion can warp more flexibly than the outer peripheral portions due to a force in the axis direction.

The back support portion may be a mesh-like member that extends from the two outer peripheral portions that sandwich the back support portion therebetween. In this manner, the back support portion can warp more flexibly than the outer peripheral portions due to a force in the axis direction.

The back support portion may be a film body that extends from the two outer peripheral portions that sandwich the back support portion therebetween. In this manner, the back support portion can warp more flexibly than the outer peripheral portions due to a force in the axis direction.

### Brief Description of Drawings

[Fig. 1] Fig. 1 is a front view illustrating an overall configuration of a medical device according to an illustrative example not falling under the scope of the claims;
[Fig. 2] Fig. 2 is an enlarged perspective view illustrating the vicinity of an expansion body;
[Fig. 3] Fig. 3 is a front view illustrating a state where one of wire portions is extended to be flat;
[Fig. 4] Fig. 4 is a view for describing a treatment method using the medical device according to the present illustrative example not falling under the scope of the claims, and is a view for schematically describing a state where the expansion body is disposed in a through-hole of an atrial septum, in which the medical device is illustrated in a front view and a biological tissue is illustrated in a cross-sectional view, respectively;
[Fig. 5] Fig. 5 is a view for schematically describing a state where a distal portion of the medical device is inserted into the atrial septum, in which a part of the medical device is illustrated in a front view, and a storage sheath and the biological tissue are illustrated in a cross-sectional view, respectively;
[Fig. 6] Fig. 6 is a view for schematically describing a state where a site on a distal side of the expansion body is developed on a left atrium side, in which the medical device is illustrated in a front view and the biological tissue is illustrated in a cross-sectional view, respectively;
[Fig. 7] Fig. 7 is a view for schematically describing a state where the expansion body is disposed in the atrial septum, in which the medical device is illustrated in a front view and the biological tissue is illustrated in a cross-sectional view, respectively;
[Fig. 8] Fig. 8 is a view for schematically describing a state where a diameter of the expansion body is increased in the atrial septum, in which the medical device is illustrated in a front view and a biological tissue is illustrated in a cross-sectional view, respectively;
[Figs. 9A and 9B] Figs. 9A and 9B are cross-sectional views illustrating the atrial septum and the expansion body: Fig. 9A illustrates a state before the atrial septum is clamped by a distal side clamping portion and a proximal side clamping portion; and Fig. 9B illustrates a state where the atrial septum is clamped by the distal side clamping portion and the proximal side clamping portion;
[Figs. 10A and 10B] Figs. 10A and 10B are cross-sectional views illustrating the atrial septum and the expansion body: Fig. 10A is a cross-sectional view taken along A-A line in Fig. 9A; and Fig. 10B is a cross-sectional view taken along B-B line in Fig. 9B;
[Fig. 11] Fig. 11 is a view for schematically describing a state where the through-hole of the atrial septum is enlarged by the expansion body, in which the medical device is illustrated in a front view and a biological tissue is illustrated in a cross-sectional view, respectively;
[Fig. 12] Fig. 12 is an enlarged perspective view illustrating the vicinity of an expansion body of a medical device according to a first modification example; and
[Figs. 13A to 13D] Figs. 13A to 13D are enlarged perspective views each illustrating the vicinity of a distal side clamping portion of a medical device according: Fig. 13A illustrates a detail of a first embodiment; Fig. 13B illustrates a detail of a second embodiment ; Fig. 13C and Fig. 13D illustrate further examples not falling under the scope of the claims.

### Description of Embodiments

Hereinafter, embodiments of this disclosure will be described with reference to the drawings. Note that, the size ratios in the drawings may be exaggerated for convenience of explanation, and may be different from the actual ratios in some cases. Moreover, in the present specification, a side of a medical device 10 to be inserted into a lumen of a living body is referred to as a "distal side", and a side at which the medical device 10 is operated is referred to as a "proximal side".

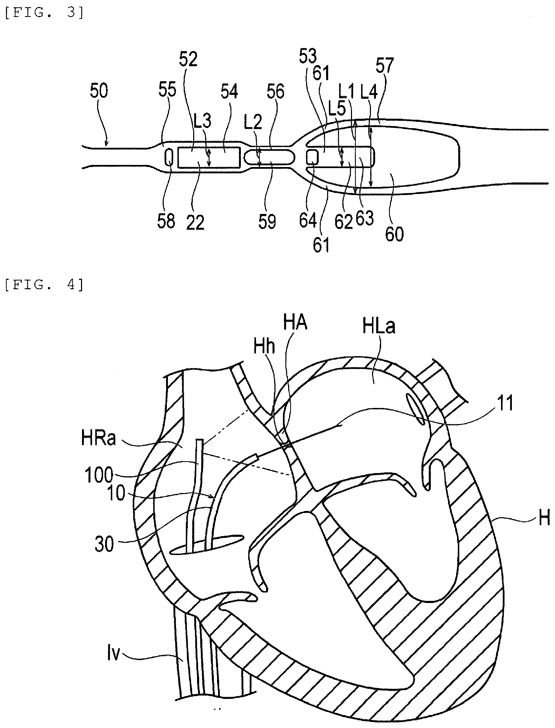

The medical device 10 according to an illustrative example not falling under the scope of the claims is configured, as shown in Fig. 4, such that a through-hole Hh formed in an atrial septum HA of a heart H of a patient is enlarged, and further, a maintenance treatment that maintains the through-hole Hh having enlarged to have the increased size can be performed.

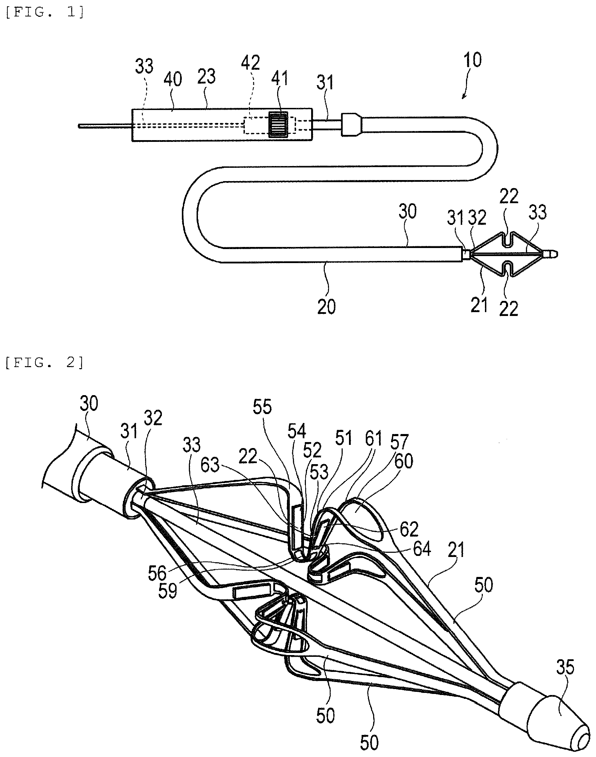

As shown in Fig. 1, the medical device 10 according to an illustrative example not falling under the scope of the claims includes an elongated shaft portion 20, an expansion body 21 that is provided in a distal portion of the shaft portion 20, and an operation unit 23 that is provided in a proximal portion of the shaft portion 20. In the expansion body 21, an energy transfer element 22 for performing the aforementioned maintenance treatment is provided.

The shaft portion 20 includes an outer shaft 31 that holds the expansion body 21 in a distal portion thereof, and a storage sheath 30 that stores the outer shaft 31. The storage sheath 30 is movable forward and rearward in an axis direction relative to the outer shaft 31. The storage sheath 30 in a state of having moved to a distal side of the shaft portion 20 can store the expansion body 21 in an inside thereof. The storage sheath 30 is moved to a proximal side in a state where the expansion body 21 is stored to enable the expansion body 21 to be exposed.

A pulling shaft 33 is stored in an inside of the outer shaft 31. The pulling shaft 33 protrudes from a distal end of the outer shaft 31 to the distal side, and has a distal portion that is fixed to a distal member 35. A proximal portion of the pulling shaft 33 is drawn out to the proximal side of the operation unit 23. The distal member 35 to which the distal portion of the pulling shaft 33 is fixed does not need to be fixed to the expansion body 21. In this manner, the distal member 35 can pull the expansion body 21 in a contracting direction. Moreover, when the expansion body 21 is stored in the storage sheath 30, the distal member 35 is separated from the expansion body 21 to the distal side, so that movement of the expansion body 21 in an extending direction becomes easy to enable the storage capability to be improved.

The operation unit 23 includes a housing 40 to be gripped by an operator, an operation dial 41 that can be rotationally operated by the operator, and a conversion mechanism 42 that is operated in conjunction with the rotation of the operation dial 41. The pulling shaft 33 is held by the conversion mechanism 42 in the inside of the operation unit 23. The conversion mechanism 42 can move the pulling shaft 33 that is held by the conversion mechanism 42 forward and rearward along the axis direction in conjunction with the rotation of the operation dial 41. As the conversion mechanism 42, for example, a rack and pinion mechanism can be used.

The expansion body 21 will be described in more details. As shown in Figs 2 and 3, the expansion body 21 includes a plurality of wire portions 50 in a circumferential direction. In the present illustrative example not falling under the scope of the claims , the four wire portions 50 are provided in the circumferential direction. Note that, the number of the wire portions 50 is not specially limited. The wire portions 50 can respectively expand and contract in a radial direction of the expansion body 21. In a natural state where no external force acts, the expansion body 21 becomes in a reference form in which the expansion body 21 is developed in the radial direction. A proximal portion of the wire portion 50 extends from a distal portion of the outer shaft 31 to the distal side. A distal portion of the wire portion 50 extends from a proximal portion of the distal member 35 to the proximal side. The wire portion 50 is inclined to increase in the radial direction from both end portions to a central portion in an axis direction of the expansion body 21. Moreover, the wire portion 50 includes a clamping portion 51 having a valley shape in an axial central portion, in the radial direction of the expansion body 21.

The clamping portion 51 includes a proximal side clamping portion 52 and a distal side clamping portion 53. The clamping portion 51 further includes a proximal side outer projection portion 55, an inner projection portion 56, and a distal side outer projection portion 57. An interval between the proximal side clamping portion 52 and the distal side clamping portion 53 is preferably opened slightly larger in the axis direction on an outer side than on an inner side in the radial direction, in the reference form. This makes it easy to dispose a biological tissue between the proximal side clamping portion 52 and the distal side clamping portion 53, from the outer side in the radial direction.

The proximal side outer projection portion 55 is positioned on a proximal side of the proximal side clamping portion 52, and is formed in a convex shape toward the outer side in the radial direction.

The inner projection portion 56 is positioned between the proximal side clamping portion 52 and the distal side clamping portion 53, and is formed in a convex shape toward the inner side in the radial direction. A central through-hole 59 is formed in the inner projection portion 56 such that the inner projection portion 56 easily bends.

The distal side outer projection portion 57 is positioned on a distal side of the distal side clamping portion 53, and is formed in a convex shape to the outer side in the radial direction. In the wire portion 50, one distal side through-hole 60 is formed in the vicinity of the distal side outer projection portion 57 and the distal side clamping portion 53. The distal side through-hole 60 penetrates into the radial direction of the expansion body 21. In this manner, the distal side outer projection portion 57 has low flexural rigidity. Therefore, the distal side outer projection portion 57 easily deforms to have a convex shape toward the outer side in the radial direction, and easily deforms such that the convex shape becomes flat. Note that, the number of the distal side through-holes 60 is not specially limited. Accordingly, the number of the distal side through-holes 60 may be two or more.

The proximal side clamping portion 52 includes a projection portion 54 that protrudes toward the distal side. In the projection portion 54, the energy transfer element 22 is disposed.

The distal side clamping portion 53 includes two outer peripheral portions 61 that are provided on both outer sides in a width direction, and a back support portion 62 that is provided between the two outer peripheral portions 61. The width direction is a direction orthogonal to the axis direction of the expansion body 21, and a direction orthogonal to the radial direction of the expansion body 21. The back support portion 62 includes a receiving surface 63 that can face the energy transfer element 22 that is disposed in the distal side clamping portion 53 when the expansion body 21 expands.

Each of the outer peripheral portions 61 has a circular arc shape that projects toward the outer side in the width direction. Therefore, between the two outer peripheral portions 61, a wide region in which the back support portion 62 and the distal side through-hole 60 are disposed can be secured. In addition, an outer side of the outer peripheral portion 61 in the width direction becomes smooth, so that the outer peripheral portion 61 is prevented from being caught on an inner surface of the storage sheath 30 that stores therein the expansion body 21. A maximum width L1 between the outer peripheral portions 61 in the width direction is larger than a maximum width L2 of the inner projection portion 56 in the width direction. Therefore, while maintaining the easiness of bending of the inner projection portion 56, the outer peripheral portions 61 can be formed in a shape expanding in the width direction. Moreover, a maximum width L5 of the receiving surface 63 in the width direction is not specially limited, but is preferably the same as or slightly larger than a maximum width L3 of the energy transfer element 22 in the width direction. In this manner, the receiving surface 63 can appropriately receive a biological tissue that is pressed by the energy transfer element 22. Moreover, the maximum width L1 between the outer peripheral portions 61 in the width direction is not specially limited, but is preferably larger than the maximum width L3 of the energy transfer element 22 in the width direction. Moreover, a maximum width L4 of the distal side through-hole 60 in the width direction is not specially limited, but is preferably larger than the maximum width L3 of the energy transfer element 22 in the width direction.

The back support portion 62 protrudes between the two outer peripheral portions 61 from a site on a side of the inner projection portion 56 of the distal side clamping portion 53 toward a side of the distal side outer projection portion 57. The back support portion 62 is disposed between the two outer peripheral portions 61 to be spaced from the two outer peripheral portions 61. An end portion of the back support portion 62 on the side of the distal side outer projection portion 57 is a free end. Accordingly, the back support portion 62 has a cantilever beam-like form in which a proximal portion thereof is fixed, and is easy to warp. Therefore, the back support portion 62 can more easily warp than each of the outer peripheral portions 61 due to a force toward the distal side that is received by the receiving surface 63. The back support portion 62 is disposed so as to be sandwiched by the two outer peripheral portions 61, but does not necessarily need to be strictly positioned in the space positioned between the two outer peripheral portions 61. The back support portion 62 may be disposed so as to be sandwiched by the two outer peripheral portions 61 at a position slightly shifted from the space positioned between the two outer peripheral portions 61. Note that, at least a part of the back support portion 62 is preferably disposed in the space positioned between the two outer peripheral portions 61.

In the back support portion 62, one back support through-hole 64 is formed on the proximal side, in other words, on a side close to the inner projection portion 56. The back support through-hole 64 penetrates into the radial direction of the expansion body 21 (the thickness direction of the wire portions 50). In this manner, the back support portion 62 has low flexural rigidity on a side close to the inner projection portion 56. Therefore, the back support portion 62 can easily warp due to a force that is received by the receiving surface 63. Note that, the number of the back support through-holes 64 is not specially limited. Accordingly, the number of the back support through-holes 64 may be two or more. Moreover, no back support through-hole 64 may be formed in the back support portion 62.

In the present illustrative example not falling under the scope of the claims, the energy transfer element 22 is provided in the proximal side clamping portion 52 and the back support portion 62 is provided in the distal side clamping portion 53, and alternatively, the energy transfer element 22 may be provided in the distal side clamping portion 53 and the back support portion 62 may be provided in the proximal side clamping portion 52.

The wire portion 50 forming the expansion body 21 has a flat plate shape cut from a cylinder, for example. The wire forming the expansion body 21 can have a thickness of 50 to 500 µm and a width of 0.3 to 2.0 mm. However, the wire forming the expansion body 21 has a size outside this range. Moreover, the shape of the wire portion 50 is not limited, and may have a circular shape in a cross section or other shapes in a cross section, for example.

The energy transfer element 22 is provided in the projection portion 54 of the proximal side clamping portion 52, so that when the clamping portion 51 clamps the atrial septum HA, the energy from the energy transfer element 22 is transferred to the atrial septum HA from the right atrium side. Note that, in a case where the energy transfer element 22 is provided in the distal side clamping portion 53, the energy from the energy transfer element 22 is transferred to the atrial septum HA from the left atrium side.

The energy transfer element 22 is configured to include, for example, a bipolar electrode that receives electric energy from an energy supply device (not illustrated) serving as an external device. In this case, electricity is supplied among the energy transfer elements 22 disposed in the respective wire portions 50. The energy transfer element 22 and the energy supply device are connected to each other by a conductive wire (not illustrated) coated with an insulating coating material. The conductive wire is drawn out to the outside via the shaft portion 20 and the operation unit 23, and is connected to the energy supply device.

Alternatively, the energy transfer element 22 may be configured as a monopolar electrode. In this case, the electricity is supplied between the energy transfer element 22 and a counter electrode plate prepared outside the body. Moreover, the energy transfer element 22 may be a heating element (electrode chip) that generates heat by receiving high-frequency electric energy from the energy supply device. In this case, electricity is supplied among the energy transfer elements 22 disposed in the respective wire portions 50. In addition, the energy transfer element 22 can be configured to include an element that can apply energy to the through-hole Hh, such as an element that provides heating or cooling operation by using microwave energy, ultrasound energy, coherent light such as laser, a heated fluid, a cooled fluid, or a chemical medium, an element that generates frictional heat, or a heater including an electric wire, and a specific form of the energy transfer element 22 is not specially limited.

The wire portion 50 can be formed of a metal material. As the metal material, for example, a titanium-based (Ti-Ni, Ti-Pd, Ti-Nb-Sn, or the like) alloy, a copper-based alloy, stainless steel, β titanium steel, or a Co-Cr alloy can be used. Note that, an alloy having a spring property such as a nickel titanium alloy may be more preferably used. However, a material for the wire portion 50 is not limited to these, and the wire portion 50 may be formed of other materials.

The shaft portion 20 includes an inner shaft 32 in the inside of the outer shaft 31, and the pulling shaft 33 is stored in an inside of the inner shaft 32. A guide wire lumen is formed in the pulling shaft 33 and the distal member 35 along the axis direction, and a guide wire 11 can be inserted through the guide wire lumen.

The storage sheath 30, the outer shaft 31, the inner shaft 32 of the shaft portion 20 are preferably formed of a material having a certain degree of flexibility. Examples of such the material can include polyolefin such as polyethylene, polypropylene, polybutene, an ethylene-propylene copolymer, an ethylene-vinyl acetate copolymer, an ionomer, or a mixture of two or more of them, soft polyvinyl chloride resin, polyamide, polyamide elastomer, polyester, polyester elastomer, polyurethane, fluorine resin such as polytetrafluoroethylene, polyimide, PEEK, silicone rubber, and latex rubber.

The pulling shaft 33 can be formed of, for example, an elongated wire material including a super elasticity alloy such as a nickel-titanium alloy and a copper-zinc alloy, a metal material such as stainless steel, and a resin material having comparatively high rigidity. Moreover, the pulling shaft 33 may be formed of the abovementioned wire material coated with a resin material such as polyvinyl chloride, polyethylene, polypropylene, an ethylene-propylene copolymer, or fluorine resin.

The distal member 35 can be formed of, for example, a super elasticity alloy such as a nickel-titanium alloy and a copper-zinc alloy, a metal material such as stainless steel, and a resin material having comparatively high rigidity.

Next, a treatment method using the medical device 10 according to the present illustrative example not falling under the scope of the claims will be described. The treatment method is performed on a patient suffering from a heart failure (left heart failure). More specifically, as shown in Fig. 4, the treatment method is performed on the patient suffering from a chronic heart failure, who has a high blood pressure in a left atrium HLa due to myocardial hypertrophy appearing in a left ventricle of the heart H and increased stiffness (hardness).

When the through-hole Hh is formed, an operator delivers an introducer in which a guiding sheath and a dilator are combined with each other to the vicinity of the atrial septum HA. The introducer can be delivered to a right atrium HRa via an inferior vena cava Iv, for example. Moreover, the introducer can be delivered using the guide wire 11. The operator can insert the guide wire 11 into the dilator, and can deliver the introducer along the guide wire 11. Note that, the insertion of the introducer, the insertion of the guide wire 11, and the like to a living body can be performed by using a publicly known method such as using an introducer for blood vessel introduction.

Next, the operator causes a puncture device (not illustrated) and the dilator to penetrate from the right atrium HRa side toward the left atrium HLa side, thereby forming the through-hole Hh. As the puncture device, for example, a device such as a wire having a sharp distal end can be used. The puncture device is inserted into the dilator, and is delivered to the atrial septum HA. The puncture device can be delivered to the atrial septum HA instead of the guide wire 11 after the guide wire 11 is extracted from the dilator.

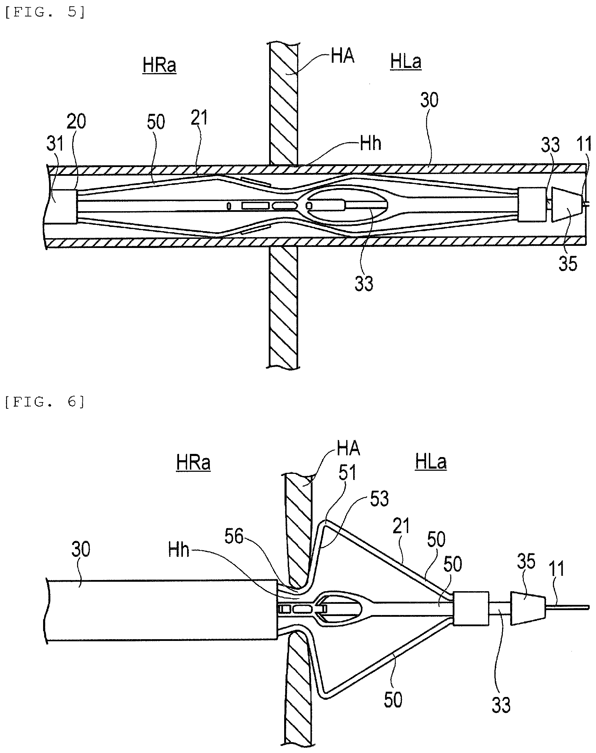

Next, as shown in Fig. 4, the operator delivers the medical device 10 to the vicinity of the atrial septum HA along the guide wire 11 inserted in advance into the left atrium HLa from the right atrium HRa via the through-hole Hh. Further, as shown in Fig. 5, a part of a distal portion of the medical device 10 passes through the through-hole Hh opened in the atrial septum HA, and reaches the left atrium HLa. When the medical device 10 is inserted, the expansion body 21 is in a contracted form in which the expansion body 21 is stored in the storage sheath 30. In the contracted form, the proximal side outer projection portion 55, the inner projection portion 56, and the distal side outer projection portion 57 having convex shapes in a natural state deforms into a shape close to a flat shape, so that the expansion body 21 contracts in the radial direction.

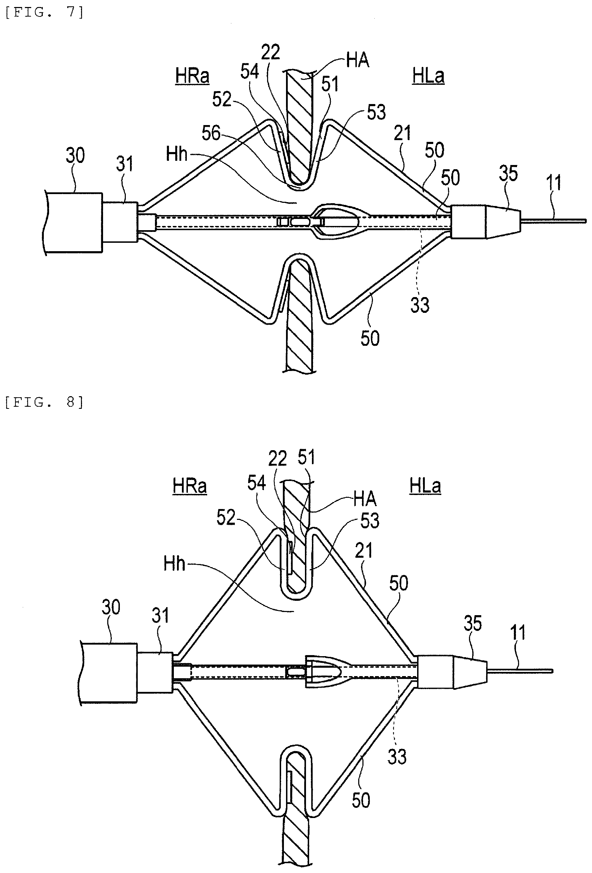

Next, as shown in Fig. 6, the storage sheath 30 is moved to the proximal side, thereby exposing a portion on a distal side of the expansion body 21 in the left atrium HLa. In this manner, the portion on the distal side in the expansion body 21 is developed in the radial direction in the left atrium HLa due to a self-restoring force. Next, as shown in Fig. 7, the storage sheath 30 is moved to the proximal side, thereby exposing the entire expansion body 21. In this manner, the portion on the proximal side in the expansion body 21 is developed in the radial direction in the right atrium HRa due to a self-restoring force. In this process, the inner projection portion 56 is disposed to an inner side of the through-hole Hh. In this manner, the entire expansion body 21 is developed due to the self-restoring force, and is recovered to an original reference form or a form close to the reference form. In this case, the atrial septum HA is disposed between the proximal side clamping portion 52 and the distal side clamping portion 53. In a clamping direction of the biological tissue, the atrial septum HA is disposed between the energy transfer element 22 and the back support portion 62. Note that, the expansion body 21 is brought into contact with the through-hole Hh, and thus is not completely returned to the reference form but may be returned to a shape close to the reference form. Note that, in this state, the expansion body 21 is not covered with the storage sheath 30, and does not receive a force from the pulling shaft 33. This form of the expansion body 21 can be defined as being included in the reference form.

Each of the outer peripheral portions 61 has a circular arc shape that projects to the outer side in the width direction. Therefore, the outer peripheral portion 61 is hard to be caught on the inner surface of the storage sheath 30. Accordingly, the expansion body 21 including the outer peripheral portions 61 is smoothly released from the storage sheath 30.

Next, the operator operates the operation unit 23 in a state where the clamping portion 51 holds the atrial septum HA, thereby moving the pulling shaft 33 to the proximal side. In this manner, as shown in Fig. 8, the expansion body 21 that receives a contracting force in the axis direction becomes in an expanded form in which the expansion body 21 has expanded more in the radial direction than in the reference form. The expansion body 21 becomes in the expanded form, so that the proximal side clamping portion 52 and the distal side clamping portion 53 come closer to each other, and the atrial septum HA is clamped between the proximal side clamping portion 52 and the distal side clamping portion 53. In this process, the energy transfer element 22 and the back support portion 62 face each other. The clamping portion 51 in the state of clamping the atrial septum HA further expands to widen the through-hole Hh in the radial direction.

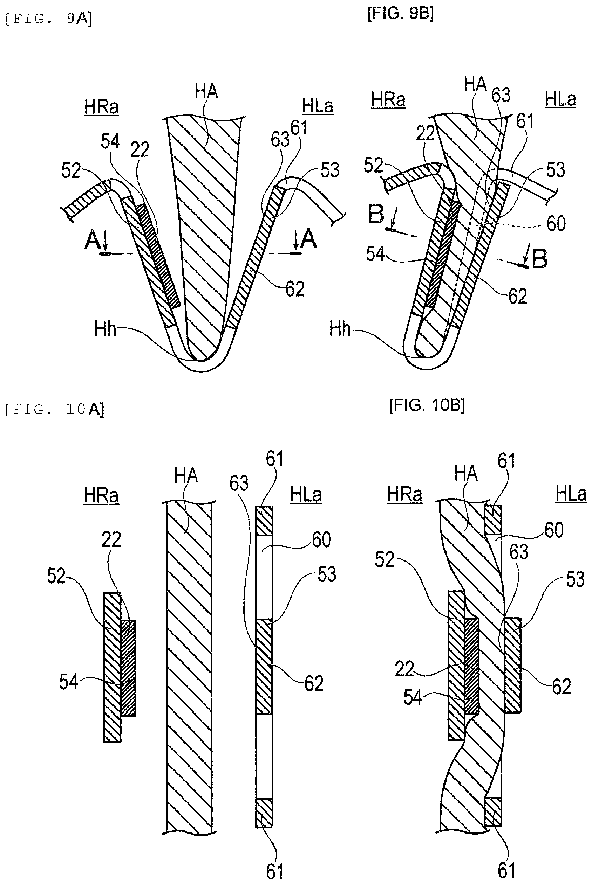

As shown in Fig. 9A and Fig. 10A, when the proximal side clamping portion 52 and the distal side clamping portion 53 come closer to each other from the state where the proximal side clamping portion 52 and the distal side clamping portion 53 are separated from each other, as shown in Fig. 9B and Fig. 10B, the atrial septum HA is clamped between the proximal side clamping portion 52 and the distal side clamping portion 53. Further, the energy transfer element 22 presses the atrial septum HA to the distal side. In this process, the distal side clamping portion 53 causes the back support portion 62 to warp to the distal side between the two outer peripheral portions 61, and receives the atrial septum HA that is pressed by the energy transfer element 22, between the two outer peripheral portions 61. The receiving surface 63 of the back support portion 62 receives a force via the atrial septum HA from the energy transfer element 22, and warps so as to be approximately parallel to the energy transfer element 22. Further, the back support portion 62 causes, while flexibly warping, a repulsion force in a reverse direction of a press-in direction of the energy transfer element 22 to act on the atrial septum HA that is pressed by the energy transfer element 22. In this manner, the energy transfer element 22 comes into close contact with the atrial septum HA. Moreover, the energy transfer element 22 can be prevented from locally floating from the atrial septum HA.

Moreover, when the energy transfer element 22 is brought into contact with the atrial septum HA, the two outer peripheral portions 61 that sandwich the back support portion 62 therebetween effectively guide the energy transfer element 22 to the back support portion 62 that is positioned between the outer peripheral portions 61. In this manner, the energy transfer element 22 can press the atrial septum HA that is supported by the two outer peripheral portions 61 while coming into contact with the atrial septum HA, and can press the atrial septum HA against the back support portion 62 that is disposed between the two outer peripheral portions 61. Therefore, the energy transfer element 22 comes into close contact with the atrial septum HA and is hard to float from the atrial septum HA, and a position of the energy transfer element 22 relative to the atrial septum HA is stably maintained between the two outer peripheral portions 61.

The atrial septum HA is clamped between the energy transfer element 22 and the back support portion 62, while being clamped in an uneven structure of the projection portion 54 and the distal side through-hole 60. In this process, the back support portion 62 warps, so that the atrial septum HA is easily clamped in the uneven structure of the projection portion 54 and the distal side through-hole 60. In this manner, the proximal side clamping portion 52 and the distal side clamping portion 53 support with each other in a circumferential direction of the expansion body 21, so that a position shift of the expansion body 21 in the circumferential direction can be suppressed therebetween. Therefore, an expanding force of the expansion body 21 can be reliably transferred to the biological tissue. The proximal side clamping portion 52 and the distal side clamping portion 53 clamp the biological tissue when the expansion body 21 expands. When the expansion body 21 expands indicates any one of the middle of the expansion of the expansion body 21, the instant when the expansion body 21 completely expands, and the time from when the expansion body 21 completely expands to when the expansion body 21 contracts.

Further, the maximum width L1 between the outer peripheral portions 61 in the width direction is larger than the maximum width L2 of the inner projection portion 56 in the width direction. Therefore, while flexibly maintaining the inner projection portion 56, a region in which the back support portion 62 is disposed is easily secured between the two outer peripheral portions 61. Moreover, the maximum width L1 between the outer peripheral portions 61 in the width direction is larger than the maximum width L3 of the energy transfer element 22 in the width direction. Therefore, the outer peripheral portions 61 easily guide the press direction of the energy transfer element 22 toward the back support portion 62. Moreover, the maximum width L4 of the distal side through-hole 60 in the width direction is larger than the maximum width L3 of the energy transfer element 22 in the width direction. Therefore, the outer peripheral portions 61 easily guide the press direction of the energy transfer element 22 toward the back support portion 62. In addition, the energy transfer element 22 easily enter between the outer peripheral portions 61, so that a force in which the proximal side clamping portion 52 including the energy transfer element 22 and the distal side clamping portion 53 including the outer peripheral portions 61 support with each other in the circumferential direction of the expansion body 21 easily acts therebetween.

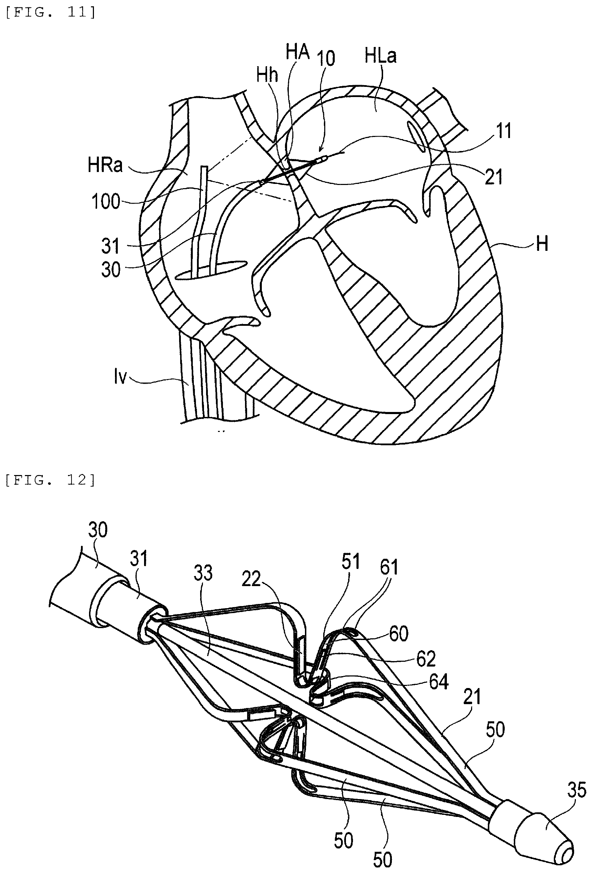

After the through-hole Hh has been enlarged, the hemodynamics is confirmed. As shown in Fig. 11, the operator delivers a hemodynamics confirming device 100 to the right atrium HRa via the inferior vena cava Iv. As the hemodynamics confirming device 100, for example, a publicly known echo catheter can be used. The operator can cause an echo image acquired by the hemodynamics confirming device 100 to be displayed on a display apparatus such as a display, and can confirm a blood volume passing through the through-hole Hh based on a displayed result.

Next, the operator performs maintenance treatment for maintaining the size of the through-hole Hh. In the maintenance treatment, energy is applied to an edge portion of the through-hole Hh through the energy transfer element 22, thereby cauterizing (heating and cauterizing) the edge portion of the through-hole Hh by using the energy. When the biological tissue in the vicinity of the edge portion of the through-hole Hh is cauterized through the energy transfer element 22, a degenerated portion having the degenerated biological tissue is formed in the vicinity of the edge portion. The biological tissue in the degenerated portion is in a state where elasticity is lost, so that the through-hole Hh can maintain a shape widened by the expansion body 21.

The medical device 10 includes the back support portion 62, so that the energy transfer element 22 comes into close contact with the atrial septum HA. Therefore, variations in the degree of cauterization by the energy transfer element 22 can be reduced. Moreover, the energy transfer element 22 can be prevented from locally floating from the atrial septum HA. Therefore, the energy transfer element 22 is prevented from supplying energy into blood and an unintended site, and generation of thrombus formation, tissue damage, and the like can be suppressed.

Moreover, the energy transfer element 22 is disposed in the projection portion 54 of the proximal side clamping portion 52. Therefore, the projection portion 54 is pressed against the atrial septum HA, whereby the maintenance treatment is performed in a state where the energy transfer element 22 is embedded in the biological tissue. In this manner, the energy transfer element 22 is prevented from supplying energy into blood and an unintended site, and generation of thrombus formation, tissue damage, and the like can be suppressed.

After the maintenance treatment, the hemodynamics are confirmed again, and in a case where the volume of blood passing through the through-hole Hh reaches a desired amount, the operator decreases the diameter of the expansion body 21, and stores the expansion body 21 in the storage sheath 30 and then extracts the expansion body 21 from the through-hole Hh. The outer peripheral portion 61 has a circular arc shape that projects to the outer side in the width direction, and thus is hard to be caught on the inner surface of the storage sheath 30. Accordingly, the expansion body 21 including the outer peripheral portions 61 is smoothly stored in the storage sheath 30. In addition, the operator extracts the entire medical device 10 to the outside of the living body, and ends the treatment.

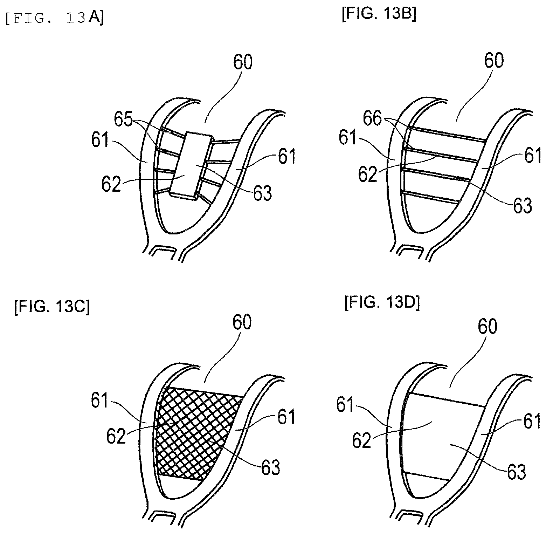

As in the foregoing, the medical device 10 according to the abovementioned illustrative example not falling under the scope of the claims includes: the elongated shaft portion 20, and the expansion body 21 that is provided in a distal portion of the shaft portion 20 and can expand and contract in a radial direction, in which: the expansion body 21 includes a plurality of wire portions 50 that are linked with the shaft portion 30, and at least one clamping portion 51 that is formed by at least one wire portion 50; the clamping portion 51 includes the energy transfer element 22 that outputs energy, and the back support portion 62; the back support portion 62 includes the receiving surface 63 that can face the energy transfer element 22 when the expansion body 21 expands; and the receiving surface 63 can be inclined so as to be approximately parallel to the energy transfer element 22 when the energy transfer element 22 moves toward the back support portion 62. In this manner, in the medical device 10, the receiving surface 63 is approximately parallel to the energy transfer element 22 in accordance with the movement of the energy transfer element 22, so that the energy transfer element 22 can come into close contact with the biological tissue between the energy transfer element 22 and the receiving surface 63. Therefore, variations in the degree of cauterization by the energy transfer element 22 can be reduced. Moreover, the energy transfer element 22 can be prevented from locally floating from the biological tissue. Therefore, the energy transfer element 22 is prevented from supplying energy into blood and an unintended site, and generation of thrombus formation, tissue damage, and the like due to the cauterization can be suppressed. In the present illustrative example not falling under the scope of the claims , the back support portion 62 is inclined by warping due to a force in the axis direction. Note that, the back support portion 62 can be inclined without warping by being supported by another deformable member, as shown in a first embodiment (see Fig. 13A), which is described later, for example.

Moreover, at least one clamping portion 51 may include the two outer peripheral portions 61 on both sides in a width direction that is a direction orthogonal to the axis direction of the expansion body 21, and a direction orthogonal to the radial direction of the expansion body 21, relative to the back support portion 62. In this manner, the outer peripheral portions 61 effectively guide the energy transfer element 22 that moves toward the back support portion 62 to the back support portion 62 that is positioned between the outer peripheral portions 61. Therefore, the energy transfer element 22 can press the biological tissue supported by the two outer peripheral portions 61, and press the biological tissue against the back support portion 62 that is disposed between the two outer peripheral portions 61. Therefore, the energy transfer element 22 comes into close contact with the biological tissue and is hard to float from the biological tissue, and the position of the energy transfer element relative to the biological tissue is stably maintained by the two outer peripheral portions 61.

Moreover, the two outer peripheral portions 61 each have a convex shape to the outer side in the width direction. In this manner, between the two outer peripheral portions 61, a wide region in which the back support portion 62 is disposed can be secured. Moreover, the two outer peripheral portions 61 in the width direction can support the biological tissue in the wide range, so that the energy transfer element 22 and the receiving surface 63 that clamp the biological tissue between the two outer peripheral portions 61 are easily maintained in the suitable positions.

Moreover, the two outer peripheral portions 61 each have a circular arc shape that smoothly projects to the outer side in the width direction. In this manner, the outer peripheral portion 61 can be stored without being caught on the inner surface of, for example, the storage sheath 30 for storing the expansion body 21 so as to be releasable. Accordingly, the outer peripheral portions 61 can be smoothly stored in the storage sheath 30, and can be smoothly released from the storage sheath 30.

Moreover, the maximum width L1 between the outer peripheral portions 61 that sandwich the back support portion 62 therebetween in the width direction is larger than the maximum width L3 of the energy transfer element 22 in the width direction. In this manner, the outer peripheral portions 61 easily guide the press direction of the energy transfer element 22 toward the back support portion 62.

Moreover, the back support portion 62 moves more than the two outer peripheral portions 61 due to a force in the axis direction to be received from the energy transfer element 22. In this manner, the back support portion 62 can flexibly receive the biological tissue that is pressed by the energy transfer element 22 while retracting more than the outer edge portion 61 in the pressing direction of the energy transfer element 22. Therefore, the energy transfer element 22 comes into close contact with the biological tissue, and is hard to float from the biological tissue.

Moreover, the expansion body 21 includes the inner projection portion 56 that projects to the inner side in the radial direction, between the energy transfer element 22 and the back support portion 62, and the maximum width L1 between the outer peripheral portions 61 that sandwich the back support portion 62 therebetween in the width direction is larger than the maximum width L2 of the inner projection portion 56 in the width direction. In this manner, while maintaining the flexibility of the inner projection portion 56, a structure in which the width between the two outer peripheral portions 61 is widened can be obtained. The inner projection portion 56 is flexible to enable the expansion body 21 to be stored in the storage sheath 30, for example. Moreover, the inner projection portion 56 is flexible to make it easy to bring the energy transfer element 22 and the back support portion 62 that sandwich the inner projection portion 56 therebetween close to or separate from each other.

Moreover, the back support portion 62 have a cantilever beam shape that extends from the wire portion 50. In this manner, the back support portion 62 can warp flexibly by receiving a force.

Moreover, this disclosure further provides a treatment method. The treatment method is a cauterization method of cauterizing a biological tissue, in which: a cauterization device is inserted into a right atrium, the cauterization device including the elongated shaft portion 20, and the expansion body 21 that is provided in a distal portion of the shaft portion 20 and can expand and contract in a radial direction, in which the expansion body 21 includes a plurality of wire portions 50 that are linked with the shaft portion 20, and at least one clamping portion 51 that is formed by at least one wire portion 50, the clamping portion 51 includes the energy transfer element 22 that outputs energy, and the back support portion 62, and the back support portion 62 includes the receiving surface 63 that can face the energy transfer element 22 when the expansion body 21 expands; a distal portion of the expansion body 21 contracted in the radial direction is inserted into the left atrium via the through-hole Hh opened in the atrial septum HA; the distal portion of the contracted expansion body 21 is developed in the left atrium due to a self-restoring force, and a proximal portion of the contracted expansion body 21 is developed in the right atrium HRa due to a self-restoring force, whereby a biological tissue in the vicinity of the edge portion of the through-hole Hh in the atrial septum HA is disposed between the energy transfer element 22 and the back support portion 62; the developed expansion body 21 is expanded in the radial direction, whereby the through-hole Hh is widened while clamping the biological tissue by the clamping portion 51; with the expansion of the through-hole Hh, the biological tissue is pressed toward the back support portion 62 by the energy transfer element 22; the receiving surface 63 of the back support portion 62 is caused to warp so as to be approximately parallel to the energy transfer element 22; a repulsion force from the receiving surface 63 of the back support portion 62 is caused to act on the biological tissue, whereby the energy transfer element 22 is brought into close contact with the biological tissue; and the biological tissue is cauterized by the energy to be output from the energy transfer element 22 having brought into close contact with the biological tissue.

In the cauterization method configured as the above, in accordance with the movement of the energy transfer element 22, the receiving surface 63 becomes approximately parallel to the energy transfer element 22, so that the energy transfer element 22 can be brought into close contact with the biological tissue between the energy transfer element 22 and the receiving surface 63. Therefore, variations in the degree of cauterization by the energy transfer element 22 can be reduced. Moreover, the energy transfer element 22 can be prevented from locally floating from the biological tissue. Therefore, the energy transfer element 22 is prevented from supplying energy into blood and an unintended site, and generation of thrombus formation, tissue damage, and the like due to the cauterization can be suppressed.

Various changes by those skilled in the art can be made within the technical scope of the present invention as defined by the claims. For example, as a first modification example shown in Fig. 12, the outer peripheral portion 61 does not need to be formed in a convex shape to the outer side in the width direction.

Moreover, the form of the back support portion 62 is not limited. For example, as the first embodiment shown in Fig. 13A, the back support portion 62 does not need to have a cantilever beam-like form, but may be supported by at least one, preferably a plurality of flexible support wires 65 that extend from the two outer peripheral portions 61. The support wire 65 is thinner and more flexible than the outer peripheral portion 61. A constituent material for the support wire 65 is not specially limited as long as it can flexibly deform, and for example, a super elasticity alloy such as a nickel-titanium alloy and a copper-zinc alloy, a metal material such as stainless steel, polyolefin such as polyethylene, polypropylene, polybutene, an ethylene-propylene copolymer, an ethylene-vinyl acetate copolymer, an ionomer, or a mixture of two or more of them, soft polyvinyl chloride resin, polyamide, polyamide elastomer, polyester, polyester elastomer, polyurethane, fluorine resin such as polytetrafluoroethylene, and a resin material such as polyimide, PEEK, silicone rubber, or latex rubber can be preferably used. The back support portion 62 is linked with the outer peripheral portions 61 only by the support wires 65. Therefore, the back support portion 62 can be inclined easier by receiving a force in the axis direction, than the outer peripheral portion 61. A plurality of the support wires 65 may be provided and disposed approximately in parallel to each other. Moreover, a plurality of the support wires 65 may be provided and disposed in a mesh shape.

Moreover, as a second embodiment shown in Fig. 13B, the back support portion 62 may include at least one, preferably a plurality of back support wires 66 having both ends that are fixed to the outer peripheral portions 61. A constituent material for the back support wire 66 is not specially limited as long as it can flexibly deform, and for example, the above-mentioned material applicable to the support wire 65 can be used suitably. The receiving surface 63 of the back support portion 62 is formed by the plurality of the back support wires 66 with gaps.

Moreover, as another illustrative example not falling under the scope of the claims and shown in Fig. 13C, the back support portion 62 may be a mesh-like member that is disposed between the two outer peripheral portions 61. The mesh-like member may include, for example, a plurality of fine lines, or a flexible member having a large number of holes being formed therein. A constituent material for the mesh-like member is not specially limited as long as it can flexibly deform, and for example, the above-mentioned material applicable to the support wire 65 can be used suitably.

Moreover, in a further illustrative example not falling under the scope of the claims shown in Fig. 13D, the back support portion 62 may be an elastically deformable film body that is disposed between the two outer peripheral portions 61. As a constituent material for the film body, for example, resin such as fluorine resin, polyethylene, and polypropylene, thermoplastic elastomer, rubber, or the like can be used suitably.

### Reference Signs List

- 10: medical device
- 20: shaft portion
- 21: expansion body
- 22: energy transfer element
- 30: shaft portion
- 50: wire portion
- 51: clamping portion
- 52: proximal side clamping portion
- 53: distal side clamping portion
- 54: projection portion
- 56: inner projection portion
- 60: distal sid-e through-hole
- 61: outer peripheral portion
- 62: back support portion
- 63: receiving surface
- 65: support wire
- 66: back support wire
- L1: maximum width between outer peripheral portions
- L2: maximum width of inner projection portion
- L3: maximum width of energy transfer element

## Claims

1. A medical device (10) comprising:
an elongated shaft portion (20, 30); and
an expansion body (21) that is provided in a distal portion of the shaft portion (20, 30), and is configured to expand and contract in a radial direction, wherein
the expansion body (21) includes a plurality of wire portions (50) that are linked with the shaft portion (20, 30), and at least one clamping portion (51) that is formed by at least one of the wire portions (50),
the clamping portion (51) includes an energy transfer element (22) that outputs energy, and a back support portion (62),
the back support portion (62) includes a receiving surface (63) that is configured to face the energy transfer element (22) when the expansion body (21) expands, and
the receiving surface (63) is configured to be inclined so as to be approximately parallel to the energy transfer element (22), when the energy transfer element (22) moves toward the back support portion (62),
wherein at least one clamping portion (51) includes two outer peripheral portions (61) on both sides in a width direction that is a direction orthogonal to an axis direction of the expansion body (21), and a direction orthogonal to the radial direction of the expansion body (21), relative to the back support portion (62), and
**characterized in that**
the back support portion (62) is a member that is supported by at least one flexible support wire (65) that extends from the two outer peripheral portions (61) that sandwich the back support portion (62) therebetween, or is at least one flexible back support wire (66) that extends from the two outer peripheral portions (61) that sandwich the back support portion (62) therebetween.

2. The medical device (10) according to Claim 1, wherein the two outer peripheral portions (61) each have a convex shape to an outer side in the width direction.

3. The medical device (10) according to Claim 2, wherein the two outer peripheral portions (61) each have a circular arc shape that smoothly projects to the outer side in the width direction.

4. The medical device (10) according to any one of Claims 1 to 3, wherein a maximum width between the two outer peripheral portions (61) that sandwich the back support portion (62) therebetween in the width direction is larger than a maximum width of the energy transfer element (22) in the width direction.

5. The medical device (10) according to any one of Claims 1 to 4, wherein the back support portion (62) moves larger than the two outer peripheral portions (61) due to a force in the axis direction to be received from the energy transfer element (22).

6. The medical device (10) according to any one of Claims 1 to 5, wherein
the expansion body (21) includes an inner projection portion (56) that projects to an inner side in the radial direction between the energy transfer element (22) and the back support portion (62), and
the maximum width between the two outer peripheral portions (61) that sandwich the back support portion (62) therebetween in the width direction is larger than a maximum width of the inner projection portion (56) in the width direction.

7. The medical device (10) according to any one of Claims 1 to 6, wherein the back support portion (62) has a cantilever beam shape that extends from the wire portion (50).

## Patentansprüche

1. Medizinische Vorrichtung (10), umfassend:
einen langgestreckten Schaftabschnitt (20, 30); und
einen Ausdehnungskörper (21), der in einem distalen Abschnitt des Schaftabschnitts (20, 30) vorgesehen und so konfiguriert ist, dass er sich in einer radialen Richtung ausdehnt und zusammenzieht, wobei
der Ausdehnungskörper (21) eine Mehrzahl von Drahtabschnitten (50), die mit dem Schaftabschnitt (20, 30) verbunden sind, und mindestens einen Klemmabschnitt (51) umfasst, der durch mindestens einen von den Drahtabschnitten (50) gebildet ist,
der Klemmabschnitt (51) ein Energieübertragungselement (22), das Energie abgibt, und einen rückwärtigen Stützabschnitt (62) umfasst,
der rückwärtige Stützabschnitt (62) eine Aufnahmefläche (63) umfasst, die so konfiguriert ist, dass sie dem Energieübertragungselement (22) zugewandt ist, wenn sich der Ausdehnungskörper (21) ausdehnt, und
die Aufnahmefläche (63) so konfiguriert ist, dass sie geneigt ist, sodass sie annähernd parallel zu dem Energieübertragungselement (22) ist, wenn sich das Energieübertragungselement (22) in Richtung des rückwärtigen Stützabschnitts (62) bewegt,
wobei mindestens ein Klemmabschnitt (51) zwei äußere Umfangsabschnitte (61) auf beiden Seiten in einer Breitenrichtung, die eine Richtung orthogonal zu einer Achsenrichtung des Ausdehnungskörpers (21) ist, und einer Richtung orthogonal zu der radialen Richtung des Ausdehnungskörpers (21) relativ zu dem rückwärtigen Stützabschnitt (62) umfasst, und
**dadurch gekennzeichnet, dass**
der rückwärtige Stützabschnitt (62) ein Element ist, das von mindestens einem flexiblen Stützdraht (65) gestützt wird, das sich von den beiden äußeren Umfangsabschnitten (61) erstreckt, die den rückwärtigen Stützabschnitt (62) zwischen sich einschließen, oder mindestens ein flexibler rückwärtiger Stützdraht (66) ist, der sich von den beiden äußeren Umfangsabschnitten (61) erstreckt, die den rückwärtigen Stützabschnitt (62) zwischen sich einschließen.

2. Medizinische Vorrichtung (10) nach Anspruch 1, wobei die beiden äußeren Umfangsabschnitte (61) jeweils eine konvexe Form zu einer Außenseite in der Breitenrichtung aufweisen.

3. Medizinische Vorrichtung (10) nach Anspruch 2, wobei die beiden äußeren Umfangsabschnitte (61) jeweils eine kreisförmige Bogenform aufweisen, die in der Breitenrichtung glatt zu der Außenseite hin vorstehen.

4. Medizinische Vorrichtung (10) nach einem der Ansprüche 1 bis 3, wobei eine maximale Breite zwischen den beiden äußeren Umfangsabschnitten (61), die den rückwärtigen Stützabschnitt (62) zwischen sich in der Breitenrichtung einschließen, größer ist als eine maximale Breite des Energieübertragungselements (22) in der Breitenrichtung.

5. Medizinische Vorrichtung (10) nach einem der Ansprüche 1 bis 4, wobei sich der rückwärtige Stützabschnitt (62) aufgrund einer Kraft in der Achsenrichtung, die von dem Energieübertragungselement (22) aufgenommen wird, stärker bewegt als die beiden äußeren Umfangsabschnitte (61) .

6. Medizinische Vorrichtung (10) nach einem der Ansprüche 1 bis 5, wobei
der Ausdehnungskörper (21) einen inneren Vorsprungsabschnitt (56) aufweist, der in radialer Richtung zwischen dem Energieübertragungselement (22) und dem rückwärtigen Stützabschnitt (62) nach innen hin vorsteht, und
die maximale Breite zwischen den beiden äußeren Umfangsabschnitten (61), die den rückwärtigen Stützabschnitt (62) zwischen sich in der Breitenrichtung einschließen, größer ist als eine maximale Breite des inneren Vorsprungsabschnitts (56) in der Breitenrichtung.

7. Medizinische Vorrichtung (10) nach einem der Ansprüche 1 bis 6, wobei der rückwärtige Stützabschnitt (62) die Form eines Auslegerbalkens aufweist, die sich von dem Drahtabschnitt (50) erstreckt.

## Revendications

1. Dispositif médical (10) comprenant :
une partie de tige (20, 30) allongée ; et
un corps d'expansion (21) qui est prévu dans une partie distale de la partie de tige (20, 30), et est configuré pour s'élargir et se contracter dans une direction radiale, dans lequel
le corps d'expansion (21) comporte une pluralité de parties de fil (50) qui sont reliées à la partie de tige (20, 30), et au moins une partie de serrage (51) qui est formée par au moins l'une des parties de fil (50),
la partie de serrage (51) comporte un élément de transfert d'énergie (22) qui délivre de l'énergie, et une partie de support dorsal (62),
la partie de support dorsal (62) comporte une surface de réception (63) qui est configurée pour faire face à l'élément de transfert d'énergie (22) lorsque le corps d'expansion (21) s'élargit, et
la surface de réception (63) est configurée pour être inclinée de manière à être approximativement parallèle à l'élément de transfert d'énergie (22), lorsque l'élément de transfert d'énergie (22) se déplace vers la partie de support dorsal (62),
dans lequel au moins une partie de serrage (51) comporte deux parties périphériques externes (61) des deux côtés dans une direction de largeur qui est une direction orthogonale à une direction d'axe du corps d'expansion (21), et une direction orthogonale à la direction radiale du corps d'expansion (21), par rapport à la partie de support dorsal (62), et
**caractérisé en ce que**
la partie de support dorsal (62) est un organe qui est supporté par au moins un fil de support flexible (65) qui s'étend à partir des deux parties périphériques externes (61) qui intercalent la partie de support dorsal (62) entre elles, ou est au moins un fil de support dorsal flexible (66) qui s'étend à partir des deux parties périphériques externes (61) qui intercalent la partie de support dorsal (62) entre elles.

2. Dispositif médical (10) selon la revendication 1, dans lequel les deux parties périphériques externes (61) ont chacune une forme convexe vers un côté externe dans la direction de largeur.

3. Dispositif médical (10) selon la revendication 2, dans lequel les deux parties périphériques externes (61) présentent chacune une forme d'arc circulaire qui fait saillie en douceur vers le côté externe dans la direction de largeur.

4. Dispositif médical (10) selon l'une quelconque des revendications 1 à 3, dans lequel une largeur maximale entre les deux parties périphériques externes (61) qui intercalent la partie de support dorsal (62) entre elles dans la direction de largeur est supérieure à une largeur maximale de l'élément de transfert d'énergie (22) dans la direction de largeur.

5. Dispositif médical (10) selon l'une quelconque des revendications 1 à 4, dans lequel la partie de support dorsal (62) se déplace plus largement que les deux parties périphériques externes (61) en raison d'une force dans la direction d'axe à recevoir de l'élément de transfert d'énergie (22).

6. Dispositif médical (10) selon l'une quelconque des revendications 1 à 5, dans lequel
le corps d'expansion (21) comporte une partie de saillie interne (56) qui fait saillie vers un côté interne dans la direction radiale entre l'élément de transfert d'énergie (22) et la partie de support dorsal (62), et
la largeur maximale entre les deux parties périphériques externes (61) qui intercalent la partie de support dorsal (62) entre elles dans la direction de largeur est supérieure à une largeur maximale de la partie de saillie interne (56) dans la direction de largeur.

7. Dispositif médical (10) selon l'une quelconque des revendications 1 à 6, dans lequel la partie de support dorsal (62) présente une forme de poutre en porte-à-faux qui s'étend à partir de la partie de fil (50).
